# FASCICULE DE BREVET EUROPEEN

(11) **EP 1 064 890 B1**
(45) Date de publication et mention de la délivrance du brevet: **14.09.2005**
(21) Numéro de dépôt: 00420137.2
(22) Date de dépôt: 26.06.2000
(51) Int. Cl.: A61F 2/40, A61F 2/30

(54) **Ensemble prothétique d'articulation de l'épaule**
Prothesenbausatz für ein Schultergelenk
Prosthetic assembly for a shoulder joint

(30) Priorité: 28.06.1999 FR 9908442
(43) Date de publication de la demande: 03.01.2001
(73) Titulaire: Aston Medical Limited, London W1R 9FB (GB); Capon, Didier, 44880 Sautron (FR); Trouilloud, Pierre, 21000 Dijon (FR); De Buttet, Michel, 59170 Croix (FR)
(72) Inventeur: Alepee, Christophe, 69003 Lyon (FR); Capon, Didier, 44880 Sautron (FR); Trouilloud, Pierre, 21000 Dijon (FR); De Buttet, Michel, 59170 Croix (FR)
(74) Mandataire: Thivillier, Patrick

(56) Documents cités:
- EP-A- 0 590 241
- EP-A- 0 776 636
- FR-A- 2 545 352
- FR-A- 2 618 065
- FR-A- 2 704 747
- US-A- 3 916 451

## Description

L'invention se rattache au secteur technique des implants orthopédiques, notamment des prothèses d'épaule.

Il est connu que, pour reproduire l'articulation de l'épaule, on peut utiliser différents types de prothèses qui comprennent essentiellement un élément huméral et un élément glénoïdien ou simplement un élément huméral dans le cas de fracture par exemple.

A partir de cette conception de base, on distingue essentiellement deux grands types d'implants orthopédiques.

Selon un premier type, l'élément huméral est fixé dans le canal médullaire de l'humérus, en étant apte à coopérer, soit directement avec la glène anatomique, soit avec un élément glénoïdien fixé au niveau de la glène de l'omoplate.

Dans le deuxième type, pour avoir une congruence parfaite humérus / glène, le principe de l'articulation est inversé en ce sens que la tête d'articulation en tant que telle est fixée au niveau de la glène pour coopérer avec une cupule de forme complémentaire que présente l'élément huméral.

L'invention concerne plus particulièrement un ensemble prothétique pour l'articulation de l'épaule conforme à la solution du deuxième type dans lequel la tête d'articulation, généralement sous forme d'un corps convexe du type hémisphérique, est rendue solidaire de la glène anatomique. Ce type d'articulation ressort par exemple de l'enseignement du brevet FR 2.618.065. Dans ce brevet, l'élément glénoïdien est constitué par une pièce évidée présentant une ouverture s'étendant sur environ les deux tiers d'une sphère et destinée à être engagée et fixée sur la glène de l'omoplate. Plus particulièrement, la pièce sphérique présente une ouverture circulaire, tandis que la glène de l'omoplate est préparée pour former un cylindre d'encastrement dans lequel est engagée ladite ouverture circulaire. L'élément huméral est constitué par une cupule sphérique de même rayon que la calotte sphérique et l'élément glénoïdien, et solidaire d'une tige d'ancrage engagée dans la diaphyse humérale.

Cette solution peut être améliorée. En effet, après mise en place de l'élément glénoïdien, il y a des risques de butée sur les ostéophytes du pilier de l'omoplate. Il peut en résulter une limitation de l'amplitude des mouvements d'adduction susceptibles d'entraîner une usure du pilier. En outre, compte tenu de la forme de l'élément glénoïdien et de son mode de fixation symétrique par rapport à la glène, des risques de butée apparaissent sous l'acromion limitant ainsi les mouvements d'abduction.

Le fait de réaliser une fixation par encastrement, comme décrit dans le brevet suscité ou d'une manière plus complexe en fixant par vis une pastille métallique sur la glène sur laquelle et solidarisée soit par vissage soit par cône autobloquant, entraîne des contraintes telles qu'il y a des débricolages à long terme.

L'invention s'est fixée pour but de remédier à ces inconvénients, de manière simple, sûre, efficace et rationnelle.

Le problème que se propose de résoudre l'invention est d'obtenir un contact de la tête d'articulation sous forme d'un corps convexe de type hémisphérique sur la glène anatomique, le plus enveloppant possible pour ne pas être limité dans les mouvements "coude au corps", c'est-à-dire les mouvements d'abduction, tout en ayant un dégagement important de l'espace sous acromial, afin d'augmenter le moment d'adduction entre le point d'attache du deltoïde et le point d'application des efforts sur la glène.

Pour résoudre un tel problème, il a été conçu et mis au point un ensemble prothétique où le centre de rotation de la tête d'articulation est décalé vers le bas par rapport au plan médian et au centre de la glène anatomique pour rabaisser, d'une manière correspondante ladite tête d'articulation qui présente, du côté du pilier externe de l'omoplate, un prolongement apte à augmenter l'amplitude de rotation en adduction, en décalant vers le haut le point d'application des efforts pour augmenter le bras de levier entre le point d'application du deltoïde qui crée le mouvement abducteur et ledit point et en dégageant l'acromion.

Pour résoudre le problème posé d'assurer l'articulation de l'élément huméral par rapport à l'élément glénoïdien,
- soit la tête d'articulation prothétique délimite une surface d'appui convexe sous forme d'une demi-sphère pleine , à rayon constant, formée à partir du prolongement ;
- soit la tête d'articulation prothétique délimite une surface d'appui convexe sous forme d'une demi-sphère pleine à rayons progressivement décroissants à partir du prolongement ;
- soit, pour une simplification d'usinage, la combinaison des deux.

Pour résoudre le problème posé d'amélioration de la fixation de la tête d'articulation par rapport à la glène anatomique, sa surface interne d'appui avec la glène prothétique, est légèrement convexe afin d'épouser au mieux la forme prothétique concave de ladite glène.

Pour résoudre le problème posé d'augmenter le mouvement d'abduction, le centre d'articulation de la tête est abaissée par rapport au centre de la glène anatomique.

Dans le cas où l'élément glénoïdien est fixé sans ciment, la surface d'appui de la tête d'articulation avec la glène anatomique, coopère avec un élément support indépendant ou platine présentant de agencements de fixation avec la glène anatomique.

L'invention est exposée ci-après plus en détail à l'aide des figures des dessins annexés dans lesquels :
- la figure 1 est une vue en perspective de la tête d'articulation destinée à être impactée au niveau de la glène ;
- la figure 2 est une vue de face de la tête d'articulation dont la surface d'appui et de glissement avec l'élément huméral, est générée par des rayons à longueur progressive sous forme d'une développante de cercle ;

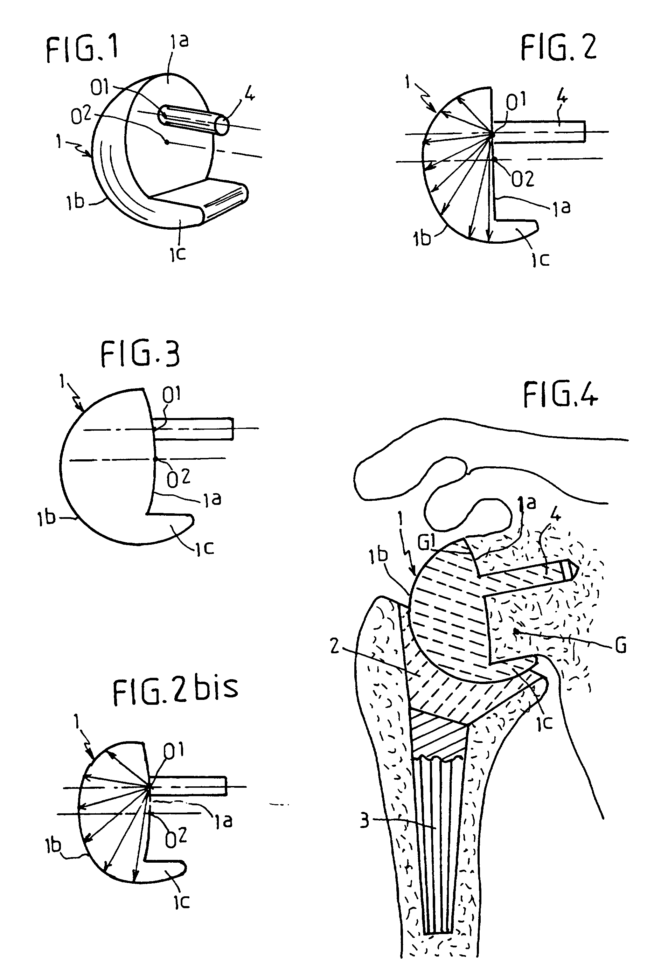

- la figure 2bis est une vue semblable à la figure 2 selon une forme de réalisation préférée au niveau de la surface d'appui avec la glène anatomique ;
- la figure 3 est une vue semblable à la figure 2bis dans une autre forme de réalisation de la tête d'articulation ;
- la figure 4 montre l'impaction de l'ensemble prothétique ;

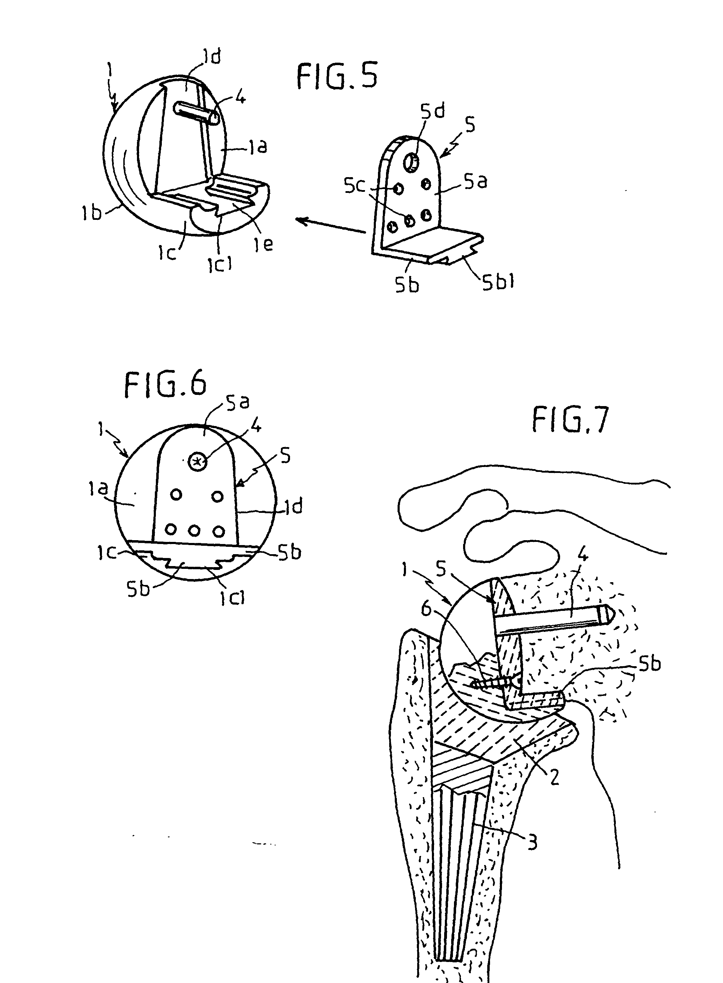

- la figure 5 est une vue en perspective, avant montage, d'une forme de réalisation de la tête d'articulation dans le cas d'une fixation sans ciment, au moyen d'un support indépendant ;
- la figure 6 montre le montage du support au niveau de la tête d'articulation;
- la figure 7 montre l'impaction de l'ensemble prothétique dans le cas d'une fixation de la tête d'articulation au moyen du support indépendant rapporté illustré figures 5 et 6 ;

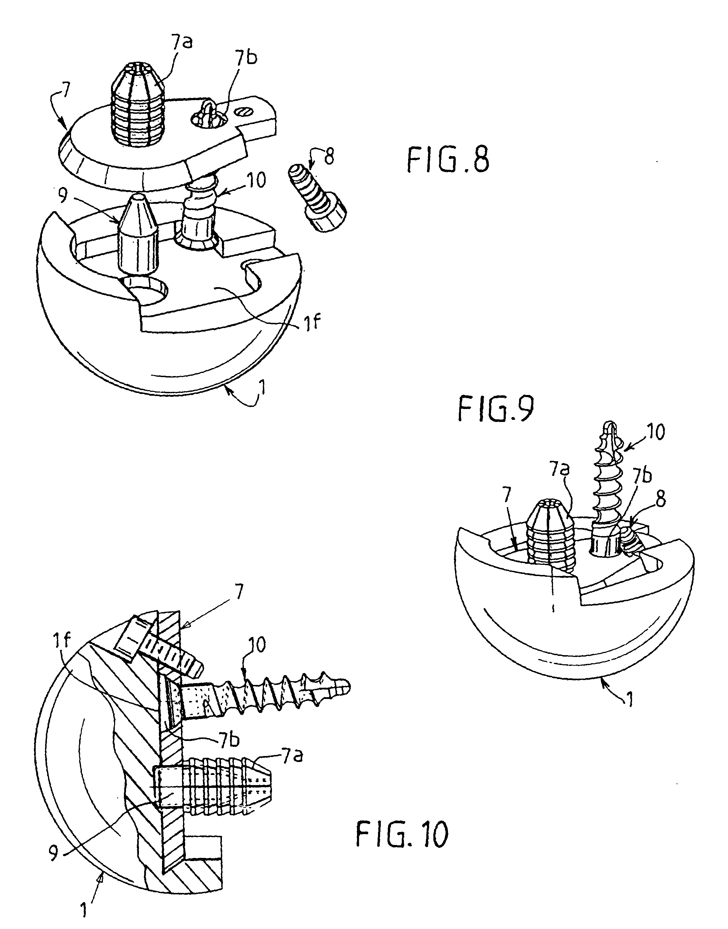

- la figure 8 est une vue en perspective d'une forme de réalisation de la tête d'articulation, notamment dans le cas d'une fixation au moyen d'un élément support indépendant, les différentes parties constitutives étant représentées avant montage ;
- la figure 9 est une vue en perspective correspondant à la figure 8 après assemblage des différentes parties constitutives ;
- la figure 10 est une vue en coupe longitudinale correspondant à la figure 9.

On rappelle, d'une manière connue, que l'ensemble prothétique de l'articulation de l'épaule, est du type de ceux comprenant un élément glénoïdien sous forme d'une tête d'articulation convexe du type hémisphérique (1) destinée à être fixée au niveau de la glène (G) de l'omoplate représentée schématiquement figure 4. La tête convexe (1) de l'élément glénoïdien coopère avec une surface d'appui et de glissement (2) que présente, directement ou d'une manière rapportée, un élément huméral (3) destiné à être fixé par tout moyen connu et approprié dans la diaphyse humérale.

La tête d'articulation (1) de l'élément glénoïdien présente une surface d'appui (1a) apte à coopérer directement ou d'une manière rapportée, avec la coupe glénoïdienne. A l'opposé, la tête d'articulation (1) présente une surface d'appui convexe (1b) qui présente, du côté du pilier externe de l'omoplate, un prolongement (1c) apte à augmenter l'amplitude de rotation. Ce prolongement (1c) est raccordé perpendiculairement à la surface d'appui (1a) en faisant ainsi office de moyen d'anti-rotation et d'anti-élévation en coopérant avec la coupe osseuse dans le pilier de l'omoplate.

Le centre de rotation de la tête d'articulation (1) est décalé vers le bas par rapport au plan médian et au centre de la glène anatomique pour rabaisser, d'une manière correspondante, la tête d'articulation.

D'une manière importante, la tête d'articulation (1) présente une tige d'ancrage (4) formée perpendiculairement à la surface (1a) et d'une manière excentrée par rapport à la surface d'appui et de glissement (1b) en étant décalée vers le haut en (0₁) au milieu de la glène anatomique centre d'application des forces, pour rabaisser, d'une manière correspondante, le centre de rotation (0₂) de la glène (1). La valeur de l'excentration est déterminée anatomiquement, afin de pouvoir obtenir une surface sphérique de contact de la cupule, avec la partie correspondante de l'élément huméral d'environ 210° et un point de rotation en surface de la glène anatomique exactement sur le Spinning Point, c'est-à-dire en bas, dans le cadran inférieur postérieur, si on découpe la glène en cadrans. Cette excentration permet en outre d'augmenter le bras de levier abducteur du deltoïde et permet de dégager l'espace sous acromiale afin d'éviter les conflits d'ostéophytes lors des positions bras levé.

On observe également que la partie de prolongement (1c) de la surface convexe déborde du plan équatorien de 180°, permettant ainsi au patient d'obtenir la position "coude au corps", et donc d'éviter toute décoadaptation de l'implant par les conflits avec les ostéophytes du pilier. Il en résulte un gain de médialisation de plusieurs millimètres au début du mouvement d'abduction, donc une augmentation du moment abducteur.

La tige ou pion d'ancrage (4) est engagée dans la masse spongieuse de la glène, et fait office de quille stabilisatrice au moment des efforts maximaux générés par les mouvements d'abduction de l'humérus.

A partir de cette conception de base, la surface convexe d'appui et de glissement peut être constituée par une demi-sphère pleine à rayon constant formé à partir du rebord (1c) (figures 4 et 7). Ou bien, dans une forme de réalisation préférée, la surface d'appui convexe (1b) est réalisée sous forme d'une demi-sphère pleine à rayons progressivement décroissants, à partir du prolongement (1c) (figure 3).

Pour des simplifications d'usinage, le fait de faire pivoter la cupule sur la tête autour du point (0₂) mais avec un point d'application des forces résultantes d'adduction en (0₁) va créer un système mécanique dans lequel le bras de levier augmente au fur et à mesure de l'adduction du membre.

La surface d'appui (1a) de la tête d'articulation (1), qui coopère avec la glène (G1) peut être plane ou, d'une manière préférée, convexe en étant bombée dans les deux plans pour avoir une congruence parfaite avec la glène de forme anatomique concave. Ainsi réalisée, la tête d'articulation glénoïdienne peut être fixée, comme indiqué précédemment, au niveau de la glène au moyen d'un ciment orthopédique.

Dans le cas d'une fixation sans ciment, la tête d'articulation glénoïdienne (1) coopère avec un élément support indépendant (5) présentant des agencements de fixation dans la glène. Plusieurs formes de réalisation peuvent être envisagées.

Dans la forme de réalisation des figures 5, 6 et 7, l'élément (5) présente deux branches en équerre (5a et 5b) de longueurs inégales et destinées à être engagées dans des entailles profilées de formes correspondantes (1d et 1e) formées respectivement dans l'épaisseur de la surface d'appui (1a) et du prolongement (1c). Par exemple, la branche (5a) destinée à coopérer avec la partie d'échancrure (1d), présente des trous (5c) pour l'engagement de vis de fixation (6) dans la glène. La branche (5b) présente un profil en section transversale en forme de queue d'aronde notamment (5b1), apte à coopérer avec un profil complémentaire (1e1) que présente l'échancrure correspondante (1e). Pour la fixation de l'élément support indépendant (5) par sa branche (5a), la branche équerrée (5b) coopère avec le pilier de l'omoplate. Il suffit ensuite d'engager la tête d'articulation prothétique sur l'élément support indépendant (5), la branche (5a) étant simplement centrée dans l'échancrure (1d). On observe que la branche (5a) présente un trou (5d) pour l'engagement de la quille de stabilité (4) de la tête d'articulation (1).

Dans la forme de réalisation illustrée figures 8, 9 et 10, l'élément support (7) se présente sous forme d'une platine de section méplate. Cette platine (7) est logée dans une empreinte complémentaire (1f) formée dans l'épaisseur de la surface d'appui (1a) de la tête d'articulation (1). Une vis (8), engagée obliquement à partir de la périphérie de la tête d'articulation (1), assure la fixation de la platine (7). Comme précédemment, la platine (7) présente les agencements de fixation avec la glène prothétique. Ainsi, la platine (7) présente, en débordement de sa face, coopérant avec la glène prothétique, un plot fendu (7a) présentant un alésage conique pour obtenir une expension diamétrale sous l'effet d'introduction d'un pion (9) que présente la tête d'articulation (1). De même, lorsque l'os est de mauvaise qualité, la platine (7) présente un trou débouchant (7b) pour l'engagement d'une vis (10) qui vient se viser au niveau du pilier coracoïdien.

Pour l'essentiel, l'élément huméral (3) demeure réalisé à la façon connue. La surface d'appui et de glissement (2) avec la tête d'articulation glénoïdienne peut être constituée par un patin en polyéthylène haute densité d'épaisseur d'environ 6 mm, et conformée pour coopérer, d'une part, avec la surface convexe (1b) de la tête (1) et, d'autre part, avec une platine humérale que présente l'élément (3). Le patin en polyéthylène peut être monté dans la platine humérale, soit d'une manière fixe, soit d'une manière mobile.

A titre indicatif, la tête d'articulation prothétique peut être réalisée en acier inoxydable M30NW ou chrome cobalt céramisé.

Les avantages ressortent bien de la description.

## Revendications

1. Ensemble prothétique d'articulation de l'épaule comprenant un élément glénoïdien sous forme d'une tête d'articulation (1) convexe du type hémisphérique présentant des agencements de fixation au niveau de la glène de l'omoplate, ladite tête coopérant avec une surface d'appui et de glissement (2) que présente un élément huméral (3) fixé dans la diaphyse humérale, **caractérisé en ce que** le centre de rotation (0₂) de la tête d'articulation (1) est décalé vers le bas par rapport au plan médian et au centre de la glène anatomique (0₁) pour rabaisser, d'une manière correspondante ladite tête d'articulation (1) qui présente, du côté du pilier externe de l'omoplate, un prolongement (1c) apte à augmenter l'amplitude de rotation en adduction, en décalant vers le haut le point d'application (0₁) des efforts pour augmenter le bras de levier entre le point d'application du deltoïde qui crée le mouvement abducteur et le dit point (0₁) et en dégageant l'acromion.

2. Ensemble selon la revendication 1, **caractérisé en ce que** la tête d'articulation (1) délimite une surface d'appui convexe (1b) sous forme d'une demi-sphère pleine, à rayon constant et de centre (0₂), formée à partir du prolongement (1c).

3. Ensemble selon la revendication 1, **caractérisé en ce que** la tête d'articulation (1) délimite une surface d'appui convexe (1b) avec l'élément huméral sous forme d'une demi-sphère pleine à rayons progressivement décroissants à partir du prolongement (1c).

4. Ensemble selon la revendication 1, **caractérisé en ce que** les agencements de fixation de la tête d'articulation (1) sont constitués par une tige d'ancrage (4) formée perpendiculairement et en débordement d'une surface d'appui (1a) avec la glène anatomique, ladite tige (4) étant décalée vers le haut du côté de l'apophyse coracoïde pour correspondre sensiblement au centre de ladite glène anatomique, point (0₁).

5. Ensemble selon la revendication 4, **caractérisé en ce que** la surface d'appui (1a) de la tête d'articulation (1) où est formée la tige d'ancrage (4), est légèrement convexe, centrée sur le point (0₁).

6. Ensemble selon l'une quelconque des revendications 1 et 4, **caractérisé en ce que** le prolongement (1c) de la tête d'articulation (1) est raccordé perpendiculairement à la surface d'appui (1a) où est formée la tige d'ancrage (4).

7. Ensemble selon la revendication 3, **caractérisé en ce que** la surface d'appui (1a) de la tête d'articulation (1) avec la glène prothétique, coopère avec un élément support indépendant ou platine (5) ou (7) présentant des agencements de fixation avec la glène prothétique.

8. Ensemble selon la revendication 7, **caractérisé en ce que** l'élément support (5) présente deux branches en équerre (5a et 5b), de longueurs inégales, engagées dans une entaille profilée d'assemblage de forme correspondante formée dans l'épaisseur de la surface d'appui (1a) de la tête d'articulation (1) et du prolongement (1c), ledit élément (5) présentant une ouverture (5d) pour le passage de la tige d'ancrage (4), la branche de longueur réduite (5b) coopérant avec le pilier de l'omoplate après conformation par sciage.

9. Ensemble selon l'une quelconque des revendications 1 à 7, **caractérisé en ce que** l'élément support (7) a une section méplate et est logé dans une entaille de forme correspondante formée dans l'épaisseur de la surface d'appui (1a) de la tête d'articulation en y étant maintenu par une vis, ledit élément support (7) présente les agencements de fixation avec la glène prothétique.

10. Ensemble selon la revendication 9, **caractérisé en ce que** l'élément support (7) présente, dans son épaisseur, un trou débouchant pour l'engagement d'une vis indépendante et/ou en débordement de sa face d'appui, des agencements sous forme d'un plot expensible (7a).

## Patentansprüche

1. Prothesenbausatz für ein Schultergelenk mit einem glenoidalen Element in Form eines konvexen Gelenkkopfes (1), der halbkugelförmig ausgebildet ist, und im Bereich der Gelenkpfanne des Schulterblattes Vorrichtungen zur Befestigung aufweist, wobei der Gelenkkopf mit einer Auflagefläche und Gleitfläche (2) zusammenwirkt, die ein zum Oberarm gehörendes Element (3) darstellen, das in der zum Oberarm gehörenden Diaphyse (Mittelstück der langen Röhrenknochen) befestigt ist, **dadurch gekennzeichnet, dass** der Drehpunkt (0₂) des Gelenkkopfes (1) in Bezug auf die Mittelebene und den Mittelpunkt der anatomischen Gelenkpfanne (0₁) nach unten verschoben ist, um den Gelenkkopf (1) entsprechend abzusenken, der auf der Seite des externen Pfeilers des Schulterblattes eine Verlängerung (1c) besitzt, welche die Drehamplitude beim Heranführen erhöhen kann, indem der Angriffspunkt (0₁) der Kräfte nach oben verschoben wird, um den Hebelarm zwischen dem Angriffspunkt des Deltoideus, der die Abduktorbewegung erzeugt, und dem Punkt (0₁) zu erhöhen, und die Schulterspitze freigegeben wird.

2. Prothesenbausatz nach Anspruch 1, **dadurch gekennzeichnet, dass** der Gelenkkopf (1) eine konvexe Auflagefläche (1b) in Form einer massiven Halbkugel mit konstantem Radius und Mittelpunkt (0₂) begrenzt, die von der Verlängerung (1c) aus gebildet wird.

3. Prothesenbausatz nach Anspruch 1, **dadurch gekennzeichnet, dass** der Gelenkkopf (1) eine konvexe Auflagefläche (1b) mit einem zum Oberarm gehörenden Element (3) in Form einer massiven Halbkugel mit allmählich abnehmendem Radius von der Verlängerung aus (1c) begrenzt.

4. Prothesenbausatz nach Anspruch 1, **dadurch gekennzeichnet, dass** die Vorrichtungen zur Befestigung des Gelenkkopfes (1) aus einem Verankerungsstab (4) bestehen, der senkrecht aus einer Auflagefläche (1a) der Gelenkpfanne hervorsteht, wobei der Stab (4) auf der Seite des Rabenschnabelfortsatzes des Schulterblattes nach oben verschoben wird, um im wesentlichen mit dem Mittelpunkt der anatomischen Gelenkpfanne, Punkt (0₁), übereinzustimmen.

5. Prothesenbausatz nach Anspruch 4, **dadurch gekennzeichnet, dass** die Auflagefläche (1a) für den Gelenkkopf (1), an der der Verankerungsstab (4) gebildet wird, leicht konvex und in Punkt (0₁) zentriert ist.

6. Prothesenbausatz nach einem der vorhergehenden Ansprüche 1 bis 4, **dadurch gekennzeichnet, dass** die Verlängerung (1c) des Gelenkkopfes (1) senkrecht mit der Auflagefläche (1a) verbunden ist, an der der Verankerungsstab (4) gebildet wird.

7. Prothesenbausatz nach Anspruch 3, **dadurch gekennzeichnet, dass** die Auflagefläche (1a) des Gelenkkopfes (1) mit der Prothesengelenkpfanne mit einem unabhängigen Trag- bzw. Stützelement oder einer Platte (5) oder (7) zusammenwirkt, welche Anordnungen zur Befestigung an der Prothesengelenkpfanne besitzt.

8. Prothesenbausatz nach Anspruch 7, **dadurch gekennzeichnet, dass** das Trag- bzw. Stützelement (5) zwei, im rechten Winkel zueinander angeordnete Schenkel (5a und 5b) unterschiedlicher Länge besitzt, welche in eine entsprechend ausgebildete, profilierte Einkerbung der Verbindung eingreifen, die in der Auflagefläche (1a) des Gelenkkopfes (1) und in der Verlängerung (1c) gebildet wird, wobei das Element (5) eine Öffnung (5d) für den Durchgang des Verankerungsstabes (4) besitzt, und der kürzere Schenkel (5b) mit dem Pfeiler des Schulterblattes zusammenwirkt, nachdem er durch Absägen entsprechend ausgebildet wurde.

9. Prothesenbausatz nach einem der vorhergehenden Ansprüche 1 bis 7, **dadurch gekennzeichnet, dass** das Trag- bzw. Stützelement (7) einen abgeflachten Querschnitt besitzt und in einer entsprechend ausgebildeten Kerbe angeordnet ist, welche sich an der Auflagefläche (1a) des Gelenkkopfes befindet und dort durch eine Schraube festgehalten wird, wobei das Trag- bzw. Stützelement (7) Anordnungen zur Befestigung an der Prothesengelenkpfanne besitzt.

10. Prothesenbausatz nach Anspruch 9, **dadurch gekennzeichnet, dass** das Trag- bzw. Stützelement (7) ein Durchgangsloch für eine unabhängige Schraube bzw. für Anordnungen in Form eines dehnbaren Stopfens besitzt, der aus seiner Auflagefläche herausragt.

## Claims

1. Prosthetic assembly for a shoulder joint, comprising a glenoid component in the form of a convex articulation head (1) of the hemispherical type with arrangements for fixing it to the glenoid cavity of the scapula, said head cooperating with a bearing and sliding surface (2) provided on a humeral component (3) fixed in the humeral diaphysis, **characterized in that** the centre of rotation (0₂) of the articulation head (1) is offset downwards in relation to the median plane and to the centre of the anatomical glenoid cavity (0₁) in order to lower, in a corresponding manner, said articulation head (1) which has, on the side of the external pillar of the scapula, an extension (1c) able to increase the extent of rotation in adduction, by upwardly offsetting the point of application (0₁) of the forces in order to increase the leverage between the point of application of the deltoid muscle which creates the abductor movement and said point (0₁), and freeing the acromion.

2. Assembly according to Claim 1, **characterized in that** the articulation head (1) delimits a convex bearing surface (1b) in the form of a solid hemisphere, with constant radius and centre (0₂), formed starting from the extension (1c).

3. Assembly according to Claim 1, **characterized in that** the articulation head (1) delimits a convex bearing surface (1b) with the humeral component in the form of a solid hemisphere with radii decreasing progressively starting from the extension (1c).

4. Assembly according to Claim 1, **characterized in that** the arrangements for fixing the articulation head (1) comprise an anchoring rod (4) formed perpendicularly and projecting from a bearing surface (1a) with the anatomical glenoid cavity, said rod (4) being offset upwards towards the coracoid process so as to correspond substantially to the centre of said anatomical glenoid cavity, point (0₁).

5. Assembly according to Claim 4, **characterized in that** the bearing surface (1a) of the articulation head (1), where the anchoring rod (4) is formed, is slightly convex, centred on the point (0₁).

6. Assembly according to either of Claims 1 and 4, **characterized in that** the extension (1c) of the articulation head (1) is joined perpendicularly to the bearing surface (1a) where the anchoring rod (4) is formed.

7. Assembly according to Claim 3, **characterized in that** the bearing surface (1a) of the articulation head (1) with the prosthetic glenoid cavity cooperates with an independent support element or plate (5) or (7) having arrangements for fixing it to the prosthetic glenoid cavity.

8. Assembly according to Claim 7, **characterized in that** the support element (5) has two branches (5a and 5b) forming an angle bracket, which branches (5a and 5b) are of unequal lengths and are engaged in a profiled recess of corresponding shape formed in the thickness of the bearing surface (1a) of the articulation head (1) and the extension (1c), said element (5) having an opening (5d) for passage of the anchoring rod (4), the branch of shorter length (5b) cooperating with the pillar of the scapula after this has been shaped by sawing.

9. Assembly according to any one of Claims 1 to 7, **characterized in that** the support element (7) has a flat cross section and is lodged in a recess of corresponding shape formed in the thickness of the bearing surface (1a) of the articulation head, and being held there by a screw, said support element (7) having the arrangements for fixing to the prosthetic glenoid cavity.

10. Assembly according to Claim 9, **characterized in that** the support element (7) has, within its thickness, a through-hole for engagement of an independent screw and/or it has, projecting from its bearing face, arrangements in the form of an expandable stud (7a).
